(19) Europäisches Patentamt
European Patent Office
Office européen des brevets

(11) Veröffentlichungsnummer: **0 449 067 A2**

(12) # EUROPÄISCHE PATENTANMELDUNG

(21) Anmeldenummer: **91104100.2**

(22) Anmeldetag: **16.03.91**

(51) Int. Cl.[5]: **C07D 249/08**, C07D 233/60, C07D 409/06, C07D 405/06, C07D 401/06, C07D 413/06, C07D 417/06, A01N 43/50, A01N 43/653

(30) Priorität: **26.03.90 DE 4009594**

(43) Veröffentlichungstag der Anmeldung: **02.10.91 Patentblatt 91/40**

(84) Benannte Vertragsstaaten: **AT BE CH DE DK ES FR GB GR IT LI NL SE**

(71) Anmelder: **BASF Aktiengesellschaft**
**Carl-Bosch-Strasse 38**
**W-6700 Ludwigshafen(DE)**

(72) Erfinder: **Seele, Rainer, Dr.**
**Leiblstrasse 3**
**W-6701 Fussgoenheim(DE)**
Erfinder: **Goetz, Norbert, Dr.**
**Schoefferstrasse 25**
**W-6520 Worms 1(DE)**
Erfinder: **Brox, Wolfgang, Dr.**
**Im Grund 13**
**W-6900 Heidelberg(DE)**
Erfinder: **Kober, Reiner, Dr.**
**20 Im Schlittweg**
**W-6701 Fussgoenheim(DE)**
Erfinder: **Ammermann, Eberhard, Dr.**
**Sachsenstrasse 3**
**W-6700 Ludwigshafen(DE)**
Erfinder: **Lorenz, Gisela, Dr.**
**Erlenweg 13**
**W-6730 Neustadt(DE)**

(54) **Fungizide Azolylethanderivate.**

(57) Azolylethanderivate der allgemeinen Formel I

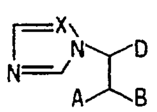

I

in welcher
A und B Alkyl, Phenyl, Biphenyl, Naphthyl, Benzyl, Cycloalkyl, Alkoxy, Alkylthio, Phenoxy, Benzyloxy, Phenylthio, Methoxycarbonylethyl oder Heteroaryl bedeuten, wobei diese Reste substituiert sein können,
D Halogen, Alkoxy, Alkylthio, Phenylthio oder Phenoxy bedeutet, wobei diese Reste substituiert sein können,
X den Rest CH oder N bedeutet,
sowie ihre pflanzenverträglichen Säureadditionssalze und Metallkomplexverbindungen und diese Verbindungen enthaltende Fungzide.

EP 0 449 067 A2

Die vorliegende Erfindung betrifft neue wertvolle Azolylethanderivate mit fungizider Wirkung, diese enthaltende Fungizide und Verfahren zur Bekämpfung von Pilzen mit diesen Verbindungen.

Es ist bekannt, Azolylethanderivative, z.B. das 1-(1,2,4-Triazol-1-yl)-1-phenylthio-2-methyl-2-phenylpropan oder das 1-(1,2,4-Triazol-1-yl)-1-(4-chlorphenylthio)-2-methyl-2-phenyl-propan (EP 91 219) oder das 1-Methoxy-1-(1,2,4-triazol-1-yl)-2-phenylethan (DE 26 40 823) als Fungizide zu verwenden. Ihre Wirkung ist jedoch unbefriedigend.

Der Erfindung lag die Aufgabe zugrunde, neue Verbindungen mit verbesserter biologischer Wirkung bereitzustellen.

Es wurde nun gefunden, daß neue Azolylethanderivate der allgemeinen Formel I

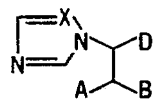

I

in welcher

A und B gleich oder verschieden sind und $C_1$-$C_8$-Alkyl, Phenyl, Biphenyl, Naphthyl, Benzyl, $C_3$-$C_6$-Cycloalkyl, $C_1$-$C_8$-Alkoxy, $C_1$-$C_8$-Alkylthio, Phenoxy, Benzyloxy, Biphenyloxy, Phenylthio, Methoxycarbonylethyl oder 5- oder 6-gliedriges Heteroaryl bedeuten, wobei diese Reste ein- bis dreifach durch Halogen, Nitro, Phenoxy, $C_1$-$C_4$-Alkyl, $C_1$-$C_4$-Alkoxy oder $C_1$-$C_4$-Halogenalkyl substituiert sein können,

mit der Maßgabe, daß mindestens einer der Substituenten A und B $C_1$-$C_8$-Alkoxy, $C_1$-$C_8$-Alkylthio, Phenoxy, Benzyloxy, Phenylthio oder 5- oder 6-gliedriges Heteroaryl bedeutet,

D Halogen, $C_1$-$C_4$-Alkoxy, $C_1$-$C_4$-Alkylthio, Phenylthio oder Phenoxy bedeutet, wobei diese Reste einfach bis dreifach durch Halogen, Nitro, Phenoxy, $C_1$-$C_4$-Alkyl oder $C_1$-$C_4$-Halogenalkyl substituiert sein können,

X den Rest CH oder N bedeutet,

sowie ihre pflanzenverträglichen Säureadditionssalze und Metallkomplexverbindungen eine bessere fungizide Wirkung besitzen als bekannte Azolverbindungen.

Im einzelnen haben die Substituenten in Formel I z.B. folgende Bedeutung:

A und B unabhängig voneinander

- unverzweigtes oder verzweigtes $C_1$-$C_8$-Alkyl, insbesondere $C_1$-$C_4$-Alkyl wie Methyl, Ethyl, Propyl, Isopropyl, Butyl, iso-Butyl, sec.-Butyl, tert.-Butyl,
- $C_3$-$c_6$-Cycloalkyl wie Cyclopropyl, Cyclopentyl, Cyclohexyl,
- Benzyl, Phenyl
- Naphthyl wie 1-Naphthyl, 2-Naphthyl,
- Biphenyl wie o-, m- oder p-Biphenyl,
- unverzweigtes oder verzweigtes $C_1$-$C_8$-Alkoxy, insbesondere $C_1$-$C_4$-Alkoxy wie Methoxy, Ethoxy, Propyloxy, Isopropyloxy, Butyloxy, iso-Butyloxy, tert.-Butyloxy,
- unverzweigtes oder verzweigtes $C_1$-$C_8$-Alkylthio, insbesondere $C_1$-$C_4$-Alkylthio wie Methylthio, Ethylthio, Propylthio, Isopropylthio, Butylthio, iso-Butylthio, sec-Butylthio,
- Benzyloxy, Phenoxy, Phenylthio,
- Methoxycarbonylethyl,
- 5- oder 6-gliedriges Heteroaryl wie Pyrrolyl, Pyrrol-2-yl, Pyrrol-3-yl, Furan-2-yl, Thien-2-yl, Thien-3-yl, Pyrid-2-yl, Pyrid-3-yl, Pyrid-4-yl, 1,3-Diazol-2-yl, Oxazol-4-yl, Oxazol-5-yl, Isoxazolyl, Isoxazol-3-yl, Isoxazol-4-yl, Isoxazol-5-yl, Thiazol-4-yl, Thiazol-5-yl, Imidazol-4-yl, 1,3-Dioxolan-2-yl.

Die genannten Reste können ein- bis dreifach substituiert sein durch

- Halogen wie Fluor, Chlor oder Brom,
- Nitro,
- Phenoxy,
- $C_1$-$C_4$-Alkyl wie oben im einzelnen genannt,
- $C_1$-$C_4$-Halogenalkyl mit 1 bis 3 Halogenatomen wie Fluor, Chlor oder Brom, z.B. Trifluormethyl, Chlorethyl, Brombutyl.

D Halogen wie Chlor oder Brom,

- unverzweigtes oder verzweigtes $C_1$-$C_4$-Alkoxy wie oben im einzelnen genannt,
- unverzweigtes oder verzweigtes $C_1$-$C_4$-Alkylthio wie oben im einzelnen genannt,
- Phenoxy
- Thiophenyl.

Die genannten Reste können ein bis dreifach substituiert sein durch

- Halogen wie Fluor, Chlor oder Brom,
- Nitro,
- Phenoxy,
- $C_1$-$C_4$-Alkyl, z.B. Methyl oder Ethyl,
- $C_1$-$C_4$-Alkoxy wie oben im einzelnen genannt,
- $C_1$-$C_4$-Halogenalkyl mit 1 bis 3 Halogenatomen wie Fluor, Chlor oder Brom, z.B. Trifluormethyl.

Die Verbindungen der Formel I enthalten asymmetrische C-Atome und können daher als Enantiomere und Diastereomere auftreten. Die Erfindung umfaßt sowohl die reinen Isomeren als auch deren Gemische. Die Gemische von Diastereomeren können nach bekannten Methoden, zum Beispiel durch fraktionierte Kristallisation oder durch Chromatographie an Kieselgel in die Komponenten getrennt werden. Die Racemate lassen sich bei den erfindungsgemäßen Verbindungen mit üblichen Methoden, beispielsweise durch Salzbildung mit einer optisch aktiven Säure, Trennung der diastereomeren Salze und Freisetzung der Enantiomeren mittels einer Base, trennen.

Als fungizide Wirkstoffe können sowohl die einzelnen Diastereomere als auch deren Gemische verwendet werden.

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß es auf das Anion i. a. nicht ankommt. Die Säureadditionssalze werden hergestellt durch Umsetzung der Azolylethanderivate (I) mit Säuren.

Metallkomplexe der Wirkstoffe I oder ihrer Salze können z.B. mit Kupfer, Zink, Zinn, Mangan, Eisen, Kobalt oder Nickel gebildet werden, indem man die Azolylethanderivate mit Metallsalzen umsetzt.

Bedeutet D in den erfindungsgemäßen Verbindungen I Chlor oder Brom, so stellt man sie z.B. auf sehr vorteilhafte Weise analog der von H. Matsumoto et al (Tetrahedron Letters 52, 5011 (1979)) beschriebenen Methode durch Umsetzung eines Aldehyds der Formel II mit einem Azol der Formel III und einem Säurehalogenid (Y $Hal_2$) entsprechend folgender Summengleichung her:

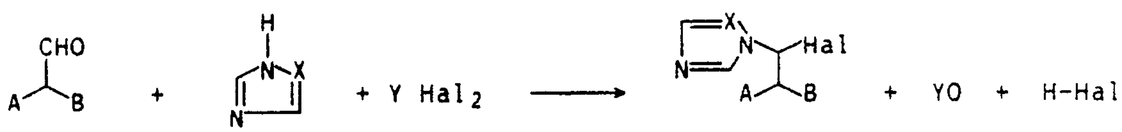

II III Ia

Die anorganischen Säurehalogenide (Y $Hal_2$) sind Halogenierungsmittel wie Phosphoroxichlorid, Thiophosgen, bevorzugt Phosgen, Thionylchlorid und -bromid.

Die Ausgangsverbindungen II sind bekannt oder in bekannter Weise erhältlich (vgl. H. Siegel, W. Himmele, Angew. Chem. 92, 182-187 (1980)).

Das Säurehalogenid wird bevorzugt in mindestens äquimolaren Mengen bezogen auf den Aldehyd II eingesetzt. Die Azolkomponente III wird z.B. in der zweifachen, bevorzugt in der 5-6-fachen molaren Menge, bezogen auf das Säurechlorid bzw. -bromid, eingesetzt.

Die Umsetzung erfolgt bevorzugt bei Temperaturen zwischen -30 und +100°C, besonders bevorzugt zwischen 0 und 20°C in Gegenwart eines Lösungsmittels.

Bevorzugte Lösungsmittel sind z.B. Nitrile wie Acetonitril, Ether wie Tetrahydrofuran, Diethylether oder Dioxan. Besonders bevorzugt werden Kohlenwasserstoffe und Chlorkohlenwasserstoffe wie Hexan, Benzol, Toluol, Methylenchlorid, Tetrachlorkohlenstoff oder Gemische der genannten Solventien.

Im allgemeinen arbeitet man bei Atmosphärendruck, sofern sich nicht wegen leichtflüchtiger Reaktionspartner ein höherer Druck, etwa bis zu 5 bar empfiehlt.

Da die Säurehalogenide und die intermediär entstehenden Zwischenprodukte hydrolyseempfindlich sind, arbeitet man bevorzugt unter Feuchtigkeitsausschluß, besonders bevorzugt in einer Schutzgasatmosphäre.

Bedeutet D in den erfindungsgemäßen Verbindungen I $C_1$-$C_4$-Alkoxy, so stellt man sie z.B. auf sehr vorteilhafte Weise analog der in DE 31 50 204 beschriebenen Methode durch Umsetzung eines Acetals der Formel IV mit einem anorganischen oder organischen Säurechlorid $R^1$-COCl und anschließend mit einem Azol der Formel III entsprechend folgender Summengleichung her:

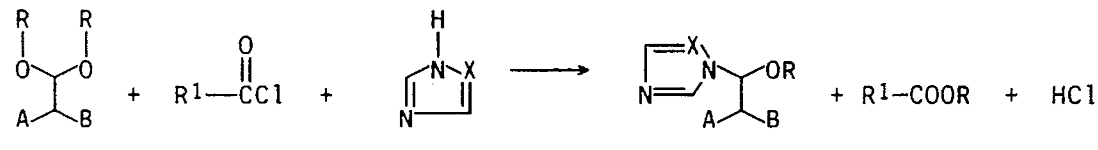

IV III

Die Ausgangsverbindungen IV sind bekannt oder in bekannter Weise durch Acetalisierung der Aldehyde II erhältlich.

Geeignete anorganische oder organische Säurehalogenide sind beispielsweise Thionylchlorid, -bromid, Acetylchlorid oder Acetylbromid. Darüber hinaus sind alle üblichen Säurehalogenide verwendbar. Das Säurehalogenid wird bevorzugt in äquimolaren Mengen bezogen auf das Acetal IV eingesetzt. Die Azolkomponente III wird z.B. in der zweifachen, bevorzugt in der 4-6fachen molaren Menge, bezogen auf das Säurehalogenid, eingesetzt.

Die Umsetzung erfolgt bei Temperaturen zwischen -30 und +100°C, besonders bevorzugt zwischen 0 und 30°C in Gegenwart eines Lösungsmittels.

Bevorzugte Lösungsmittel sind z.B. Ketone wie Aceton, Methylethylketon oder Cyclohexanon, Nitrile wie Acetonitril oder Propionitril, Alkohole wie Methanol, Ethanol, i-Propanol, n-Butanol, Glykole, Ester wie Essigsäuremethylester, Essigsäureethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methyl-pyrrolidon, ferner Dimethylsulfoxid, Sulfolan oder Gemische der genannten Lösungsmittel.

Da die Säurehalogenide und die intermediär entstehenden Zwischenprodukte hydrolyseempfindlich sind, arbeitet man bevorzugt unter Feuchtigkeitsausschluß, besonders bevorzugt in einer Schutzgasatmosphäre.

Die Verbindungen I, in denen D einen anderen Substituenten als Chlor, Brom oder $C_1$-$C_4$-Alkoxy bedeutet, lassen sich besonders vorteilhaft aus den Chlor- oder Bromverbindungen Ia herstellen, indem man diese mit einer Verbindung H-D sowie einer Base umsetzt.

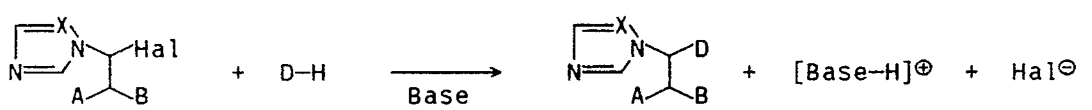


Ia I

Die Komponente H-D wird vorteilhaft in stöchiometrischen Mengen, bevorzugt in etwa 20 %igem Überschuß eingesetzt, bezogen auf das Azolylethanderivat Ia.

Die Reaktion erfolgt vorteilhaft unter Zusatz einer organischen oder anorganischen Hilfsbase und/oder eines Reaktionsbeschleunigers in Gegenwart eines Lösungsmittels.

Die Menge an Base und Reaktionsbeschleuniger kann je nach der eingesetzten Verbindung variiert werden. Vorteilhaft setzt man einen geringen überschuß an Base, bezogen auf das Azolylethanderivat Ia, ein.

Geeignete Basen sind beispielsweise Alkalihydroxide wie Lithium-, Natrium- oder Kaliumhydroxid, Alkalicarbonate wie Natrium- oder Kaliumcarbonat oder Natrium- oder Kaliumhydrogencarbonat, Alkaliamide wie Natrium- oder Kaliumamid sowie die organischen Basen Pyridin, 4-Dialkylaminopyridin, Dialkylamine und Dialkylaniline oder bevorzugt das Alkalisalz der Komponente H-D.

Der Reaktionsbeschleuniger wird bevorzugt in katalytischen Mengen zum Reaktionsgemisch gegeben.

Als Reaktionsbeschleuniger können z.B. Metallhalogenide, bevorzugt Natriumjodid oder Kaliumjodid, quartäre Ammoniumsalze wie Tetraalkylammoniumchlorid,-bromid oder -jodid, Aryltrialkylammoniumhalogenide wie Benzyltriethylammoniumchlorid oder -bromid und Kronenether wie 12-Krone-4, 15-Krone-5, Benzo-15-Krone-5, Dibenzo-18-Krone-6 oder Dicyclohexano-18-Krone-6, verwendet werden.

Als Lösungsmittel werden bevorzugt Ketone wie Aceton, Methylethylketon oder Cyclohexanon, Nitrile wie Acetonitril oder Propionitril, Alkohole wie Methanol, Ethanol, i-Propanol, n-Butanol, Glykole, Ester wie Essigsäuremethylester, Essigsäureethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder Gemische der genannten Lösungsmittel verwendet.

Die Umsetzung wird vorteilhaft bei Temperaturen zwischen 0 und 180°C, bevorzugt bei der Siedetemperatur des verwendeten Lösungsmittels, durchgeführt.

Die erfindungsgemäßen Verfahren zur Herstellung von Azolylethanderivaten können kontinuierlich oder diskontinuierlich durchgeführt werden.

Die nachfolgenden Beispiele erläutern die Herstellung der Wirkstoffe.

Herstellungsbeispiele

Beispiel 1
1-Chlor-1-(1,2,4-Triazol-1-yl)-2-(4-methylphenoxy)-propan

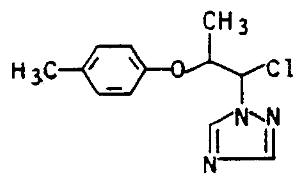

Eine Lösung aus 54,5 g (0,79 mol) Triazol in 150 ml Methylenchlorid wurde bei 0°C unter Stickstoffatmosphäre mit 23,5 g (0,2 mol) Thionylchlorid und nach 30 minütigem Rühren bei 25°C mit 20 g 2-(4-Methylphenoxy)-propanal versetzt.

Nach 12-stündiger Reaktionszeit bei 25°C wurden 100 ml Wasser zugegeben, wonach die abgetrennte wäßrige Phase zweimal mit Methylenchlorid extrahiert wurde. Die vereinigten organischen Phasen wurden sodann wie üblich auf das Triazolderivat aufgearbeitet.
Ausbeute: 25,5 g (84 %)

Beispiel 2
1-Phenylthio-1-(1,2,4-triazol-1-yl)-2-(4-methylphenoxy)-propan

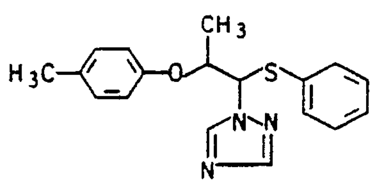

Eine Lösung aus 4,0 g Natriumhydrid (80 %ige (Gew.-%) Suspension in Mineralöl) und 100 ml N,N-Dimethylformamid wurde bei 25°C langsam mit 13 g (0,12 mol) Thiophenol und nach 30 minütigem Rühren mit 19,8 g (0,08 mol) 1-Chlor-1-(1,2,4-triazol-1-yl)-2-(4-methylphenoxy)-propan versetzt. Nach 24-stündiger Reaktionszeit bei 25°C wurden 100 ml Wasser zugegeben. Nach Extraktion mit Methyl-tert.-butylether wurde die organische Phase gewaschen und wie üblich aufgearbeitet.
Ausbeute: 18,7 g (72 %)
Entsprechend den Beispielen 1 und 2 wurden die in der Tabelle aufgeführten Verbindungen hergestellt.

Tabelle

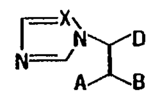

| Beispiel | A | B | D | X | Schmp/IR[$cm^{-1}$] | Isomer |
|---|---|---|---|---|---|---|
| 1 | $CH_3$ | 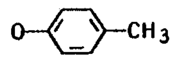 | Cl | N | 1509, 1276, 1232, 1135, 815 | $D_1$:$D_2$=2:1 |
| 2 | $CH_3$ | 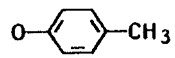 | 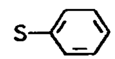 | N | Harz | $D_1$:$D_2$=2:1 |
| 3 | $CH_3$ | 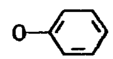 | Cl | N | | |
| 4 | $CH_3$ | 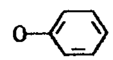 | Br | N | | |
| 5 | $CH_3$ | 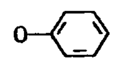 | 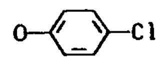 | N | | |
| 6 | $CH_3$ | 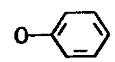 | $OCH_3$ | N | | |
| 7 | $CH_3$ | 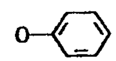 | $OC_2H_5$ | N | | |
| 8 | $CH_3$ | 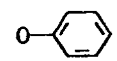 | $SCH_3$ | N | | |
| 9 | $CH_3$ | 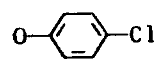 | Cl | N | Harz | $D_1$:$D_2$=2:1 |
| 10 | $CH_3$ | 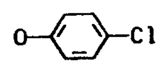 | $OCH_3$ | N | | |
| 11 | $CH_3$ | 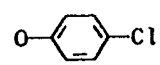 | $OC_2H_5$ | N | | |
| 12 | $CH_3$ | 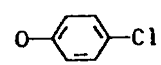 | 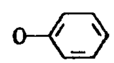 | N | Harz | $D_1$:$D_2$=2:1 |
| 13 | $CH_3$ | 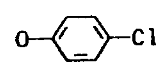 | 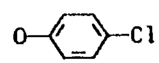 | N | | |

| Bei-spiel | A | B | D | X | Schmp/IR[$cm^{-1}$] | Isomer |
|---|---|---|---|---|---|---|
| 14 | $CH_3$ | 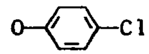 | 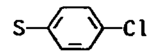 | N | | |
| 15 | $CH_3$ | 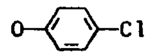 | 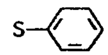 | N | | |
| 16 | $CH_3$ | 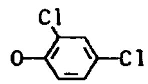 | Cl | N | 1479,1277,1262,1135,802 | $D_1$:$D_2$=3:2 |
| 17 | $CH_3$ | 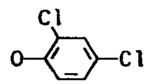 | $OCH_3$ | N | | |
| 18 | $CH_3$ | 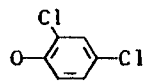 | $OC_2H_5$ | N | | |
| 19 | $CH_3$ | 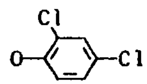 | 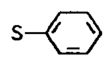 | N | | |
| 20 | $CH_3$ | 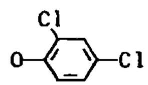 | 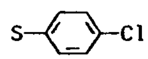 | N | | |
| 21 | $CH_3$ | 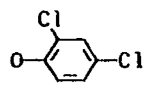 | 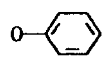 | N | | |
| 22 | $CH_3$ | 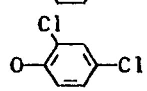 | 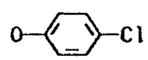 | N | | |
| 23 | $CH_3$ | 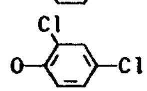 | 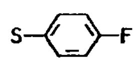 | N | | |
| 24 | $CH_3$ | 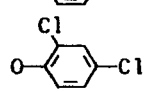 | 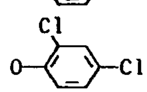 | N | | |
| 25 | $CH_3$ | 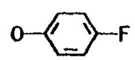 | Cl | N | | |

| Beispiel | A | B | D | X | Schmp/IR[$cm^{-1}$] | Isomer |
|---|---|---|---|---|---|---|
| 26 | $CH_3$ | 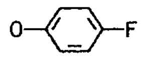 | $OCH_3$ | N | | |
| 27 | $CH_3$ | 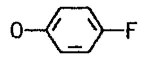 | 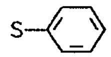 | N | | |
| 28 | $CH_3$ | 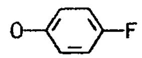 | 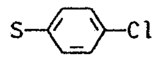 | N | | |
| 29 | $CH_3$ | 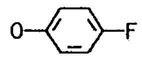 | 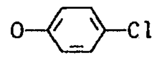 | N | | |
| 30 | $CH_3$ | 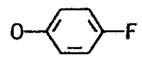 | 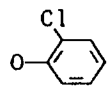 | N | | |
| 31 | $CH_3$ | 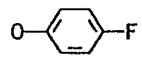 | 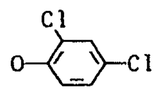 | N | | |
| 32 | $CH_3$ | 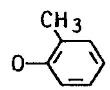 | Cl | N | Harz | $D_1:D_2=3:1$ |
| 33 | $CH_3$ | 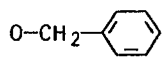 | Cl | N | 1505, 1276, 1136, 1100, 741, 698 | $D_1:D_2=6:5$ |
| 34 | $CH_3$ | 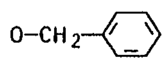 | $OCH_3$ | N | | |
| 35 | $CH_3$ | 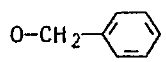 | $OC_2H_5$ | N | | |
| 36 | $CH_3$ | 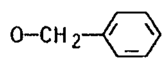 | $SCH_3$ | N | | |
| 37 | $CH_3$ | 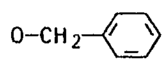 | 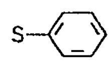 | N | | |
| 38 | $CH_3$ | 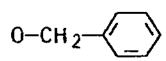 | 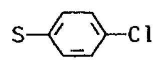 | N | | |
| 39 | $CH_3$ | 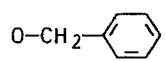 | 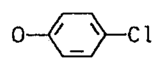 | N | | |

| Beispiel | A | B | D | X | Schmp/IR[$cm^{-1}$] | Isomer |
|---|---|---|---|---|---|---|
| 40 | $CH_3$ | $O-CH_2-$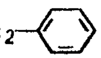 | 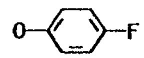 | N | | |
| 41 | $CH_3$ | $OCH_3$ | Cl | N | | |
| 42 | $CH_3$ | $OCH_3$ | 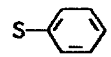 | N | | |
| 43 | $CH_3$ | $OCH_3$ | 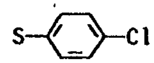 | N | | |
| 44 | $CH_3$ | $OCH_3$ | 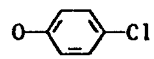 | N | | |
| 45 | $CH_3$ | $OCH_3$ | 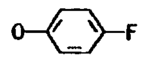 | N | | |
| 46 | $CH_3$ | $OCH_3$ | 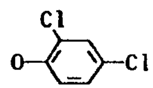 | N | | |
| 47 | $CH_3$ | $OC_2H_5$ | Cl | N | | |
| 48 | $CH_3$ | $O_2CH_5$ | 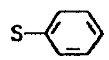 | N | | |
| 49 | $CH_3$ | $O_2CH_5$ | 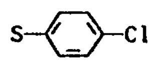 | N | | |
| 50 | $CH_3$ | $O_2CH_5$ | 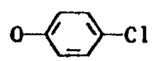 | N | | |
| 51 | $CH_3$ | $O_2CH_5$ | 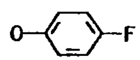 | N | | |
| 52 | $CH_3$ | $OC_3H_7$ | Cl | N | | |
| 53 | $CH_3$ | $OC_3H_7$ | 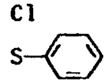 | N | | |
| 54 | $CH_3$ | $OC_3H_7$ | 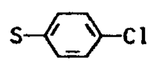 | N | | |
| 55 | $CH_3$ | $OC_3H_7$ | 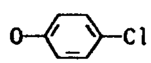 | N | | |

| Beispiel | A | B | D | X | Schmp/IR[$cm^{-1}$] | Isomer |
|---|---|---|---|---|---|---|
| 56 | $CH_3$ | $OC_3H_7$ | 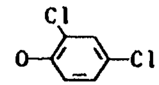 | N | | |
| 57 | $CH_3$ | $OC_4H_9$ | Cl | N | | |
| 58 | $CH_3$ | $OC_4H_9$ | 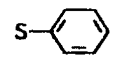 | N | | |
| 59 | $CH_3$ | $OC_4H_9$ | 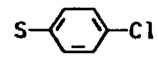 | N | | |
| 60 | $CH_3$ | $OC_4H_9$ | 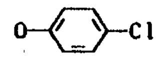 | N | | |
| 61 | $CH_3$ | $OC_4H_9$ | 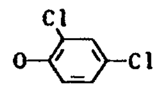 | N | | |
| 62 | $CH_3$ | $S-CH_3$ | Cl | N | | |
| 63 | $CH_3$ | $S-CH_3$ | 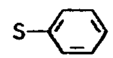 | N | | |
| 64 | $CH_3$ | $S-CH_3$ | 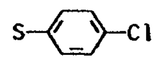 | N | | |
| 65 | $CH_3$ | $S-CH_3$ | 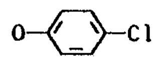 | N | | |
| 66 | $CH_3$ | $S-C_2H_5$ | Cl | N | | |
| 67 | $CH_3$ | $S-C_2H_5$ | 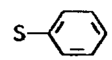 | N | | |
| 68 | $CH_3$ | $S-C_2H_5$ | 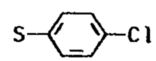 | N | | |
| 69 | $CH_3$ | $S-C_2H_5$ | 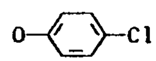 | N | | |
| 70 | $CH_3$ | 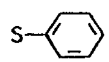 | Cl | N | | |
| 71 | $CH_3$ | 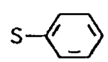 | 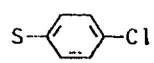 | N | | |
| 72 | $CH_3$ | 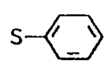 | 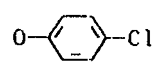 | N | | |

| Beispiel | A | B | D | X | Schmp/IR[$cm^{-1}$] | Isomer |
|---|---|---|---|---|---|---|
| 73 | $CH_3$ | 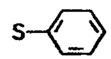 | 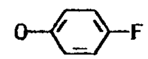 | N | | |
| 74 | $CH_3$ | 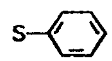 | 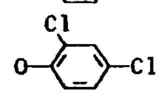 | N | | |
| 75 | H | $C_6H_5$ | Cl | N | | |
| 76 | H | $4-Cl-C_6H_4$ | Cl | N | | |
| 77 | H | $2,4-Cl_2-C_6H_3$ | Cl | N | | |
| 78 | H | $2-Cl-C_6H_4$ | Cl | N | | |
| 79 | H | $4-F-C_6H_4$ | Cl | N | Harz | – |
| 80 | H | $4-F-C_6H_4$ | 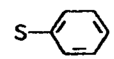 | N | 1510, 1274, 1224, 1135, 748 | |
| 81 | H | $4-CH_3-C_6H_4$ | Cl | N | | |
| 82 | H | 2-Thienyl | Cl | N | | |
| 83 | H | 3-Thienyl | Cl | N | | |
| 84 | H | 2-Furyl | Cl | N | | |
| 85 | $C_2H_5$ | $O-C_6H_5$ | Cl | N | | |
| 86 | $C_2H_5$ | $O_3C_6H_5$ | $SC_6H_5$ | N | | |
| 87 | $C_2H_5$ | $O-C_6H_5$ | 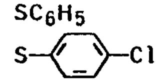 | N | | |
| 88 | $C_2H_5$ | 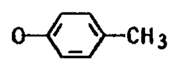 | Cl | N | | |
| 89 | $C_2H_5$ | 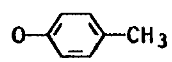 | $SC_6H_5$ | N | | |
| 90 | $C_2H_5$ | 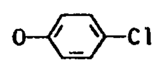 | Cl | N | | |
| 91 | $C_2H_5$ | 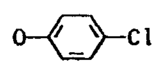 | $SC_6H_5$ | N | | |

| Beispiel | A | B | D | X | Schmp/IR[$cm^{-1}$] | Isomer |
|---|---|---|---|---|---|---|
| 92 | $CH_3$ | 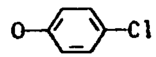 | 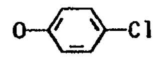 | N | | |
| 93 | $C_2H_5$ | 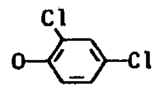 | Cl | N | | |
| 94 | $C_2H_5$ | 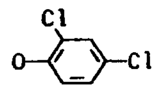 | $SC_6H_5$ | N | | |
| 95 | $C_2H_5$ | 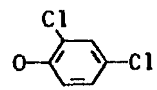 | 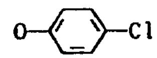 | N | | |
| 96 | $C_2H_5$ | 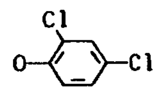 | 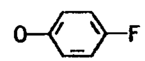 | N | | |
| 97 | $C_2H_5$ | 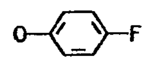 | Cl | N | | |
| 98 | $C_2H_5$ | 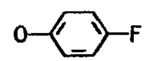 | 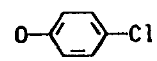 | N | | |
| 99 | $C_2H_5$ | 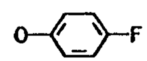 | 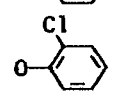 | N | | |
| 100 | $C_2H_5$ | 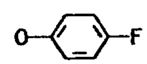 | 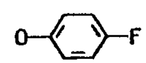 | N | | |
| 101 | $C_2H_5$ | $OCH_2-C_6H_5$ | Cl | N | | |
| 102 | $C_2H_5$ | $OCH_3$ | Cl | N | | |
| 103 | $C_2H_5$ | $OCH_3$ | 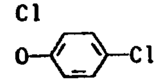 | N | | |
| 104 | $C_2H_5$ | $OCH_3$ | 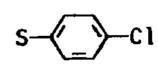 | N | | |
| 105 | $C_2H_5$ | $OCH_3$ | $SC_6H_5$ | N | | |
| 106 | $C_2H_5$ | $OC_2H_5$ | Cl | N | | |

| Bei-spiel | A | B | D | X | Schmp/IR[$cm^{-1}$] | Isomer |
|---|---|---|---|---|---|---|
| 107 | $C_2H_5$ | $OC_3H_7$ | Cl | N | | |
| 108 | $C_2H_5$ | $SCH_3$ | Cl | N | | |
| 109 | $C_2H_5$ | $SC_2H_5$ | Cl | N | | |
| 110 | $C_2H_5$ | $SC_6H_5$ | Cl | N | | |
| 111 | $C_2H_5$ | $SC_6H_5$ | $OC_6H_5$ | N | | |
| 112 | $C_2H_5$ | $SC_6H_5$ | 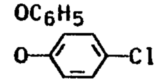 | N | | |
| 113 | $C_2H_5$ | $SC_6H_5$ | 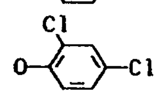 | N | | |
| 114 | $C_2H_5$ | $SC_6H_5$ | 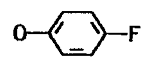 | N | | |
| 115 | $C_2H_5$ | $SC_6H_5$ | $SC_6H_5$ | N | | |
| 116 | $C_2H_5$ | $SC_6H_5$ | 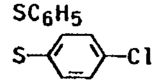 | N | | |
| 117 | $C_3H_7$ | $OC_6H_5$ | Cl | N | | |
| 118 | $C_3H_7$ | $OC_6H_5$ | $SC_6H_5$ | N | | |
| 119 | $C_3H_7$ | $OC_6H_5$ | 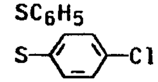 | N | | |
| 120 | $C_3H_7$ | 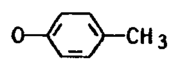 | Cl | N | | |
| 121 | $C_3H_7$ | 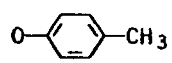 | $SC_6H_5$ | N | | |
| 122 | $C_3H_7$ | 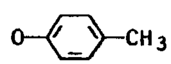 | 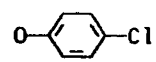 | N | | |
| 123 | $C_3H_7$ | 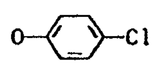 | Cl | N | | |
| 124 | $C_3H_7$ | 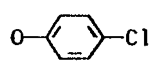 | $SC_6H_5$ | N | | |

| Beispiel | A | B | D | X | Schmp/IR[$cm^{-1}$] | Isomer |
|---|---|---|---|---|---|---|
| 125 | $C_3H_7$ | 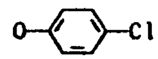 | 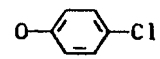 | N | | |
| 126 | $C_3H_7$ | 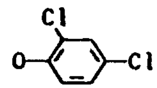 | Cl | N | | |
| 127 | $C_3H_7$ | 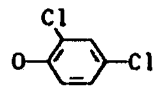 | 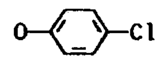 | N | | |
| 128 | $C_3H_7$ | 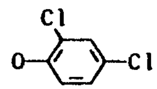 | 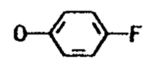 | N | | |
| 129 | $C_3H_7$ | 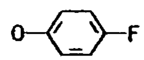 | Cl | N | | |
| 130 | $C_3H_7$ | 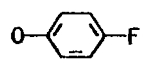 | 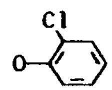 | N | | |
| 121 | $C_3H_7$ | 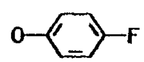 | 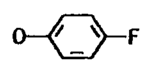 | N | | |
| 132 | $C_3H_7$ | $OCH_2C_6H_5$ | Cl | N | | |
| 133 | $C_3H_7$ | $OCH_3$ | Cl | N | | |
| 134 | $C_3H_7$ | $OCH_3$ | 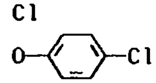 | N | | |
| 135 | $C_3H_7$ | $OCH_3$ | 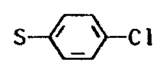 | N | | |
| 136 | $C_3H_7$ | $OCH_3$ | $SC_6H_5$ | N | | |
| 137 | $C_3H_7$ | $OC_2H_5$ | Cl | N | | |
| 138 | $C_3H_7$ | $OC_3H_7$ | Cl | N | | |
| 139 | $C_3H_7$ | $SCH_3$ | Cl | N | | |
| 140 | $C_3H_7$ | $SC_2H_5$ | Cl | N | | |
| 141 | $C_3H_7$ | $SC_2H_5$ | $OC_6H_5$ | N | | |

| Beispiel | A | B | D | X | Schmp/IR[$cm^{-1}$] | Isomer |
|---|---|---|---|---|---|---|
| 142 | $C_3H_7$ | $SC_2H_5$ | 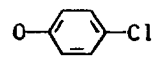 | N | | |
| 143 | $C_3H_7$ | $SC_2H_5$ | 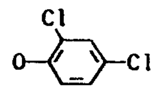 | N | | |
| 144 | $C_3H_7$ | $SC_2H_5$ | 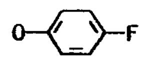 | N | | |
| 145 | $C_3H_7$ | $SC_2H_5$ | $SC_6H_5$ | N | | |
| 146 | $C_3H_7$ | $SC_2H_5$ | 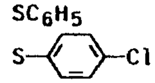 | N | | |
| 147 | $C_3H_7$ | $SC_6H_5$ | Cl | N | | |
| 148 | $C_3H_7$ | $SC_6H_5$ | $OC_6H_5$ | N | | |
| 149 | $C_3H_7$ | $SC_6H_5$ | 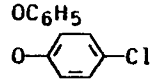 | N | | |
| 150 | $C_3H_7$ | $SC_6H_5$ | 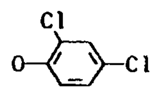 | N | | |
| 151 | $C_3H_7$ | $SC_6H_5$ | 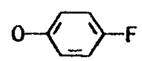 | N | | |
| 152 | $C_3H_7$ | $SC_6H_5$ | $SC_6H_5$ | N | | |
| 153 | $C_4H_9$ | $OC_6H_5$ | Cl | N | | |
| 154 | $C_4H_9$ | 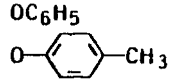 | Cl | N | | |
| 155 | $C_4H_9$ | 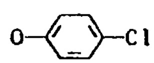 | Cl | N | | |
| 156 | $C_4H_9$ | 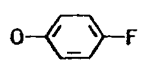 | Cl | N | | |
| 157 | $C_4H_9$ | 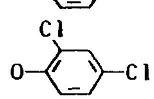 | Cl | N | | |

| Beispiel | A | B | D | X | Schmp/IR[$cm^{-1}$] | Isomer |
|---|---|---|---|---|---|---|
| 158 | $C_4H_9$ | $OCH_2C_6H_5$ | Cl | N | | |
| 159 | $C_4H_9$ | $OCH_3$ | Cl | N | | |
| 160 | $C_4H_9$ | $OC_3H_7$ | Cl | N | | |
| 161 | $C_4H_9$ | $SCH_3$ | Cl | N | | |
| 162 | $C_4H_9$ | $SC_6H_5$ | Cl | N | | |
| 163 | $C_6H_5$ | $OC_6H_5$ | Cl | N | | |
| 164 | $C_6H_5$ | 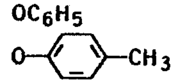 | Cl | N | | |
| 165 | $C_6H_5$ | 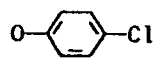 | Cl | N | | |
| 166 | $C_6H_5$ | 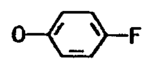 | Cl | N | | |
| 167 | $C_6H_5$ | 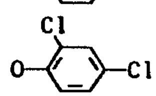 | Cl | N | | |
| 168 | $C_6H_5$ | $OCH_3$ | Cl | N | | |
| 169 | $C_6H_5$ | $SCH_3$ | Cl | N | | |
| 170 | $C_6H_5$ | $SC_6H_5$ | Cl | N | | |
| 171 | 4-F-$C_6H_4$ | $OC_6H_5$ | Cl | N | | |
| 172 | 4-F-$C_6H_4$ | 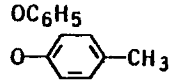 | Cl | N | | |
| 173 | 4-F-$C_6H_4$ | 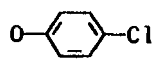 | Cl | N | | |
| 174 | 4-F-$C_6H_4$ | 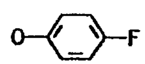 | Cl | N | | |
| 175 | 4-F-$C_6H_4$ | 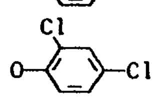 | Cl | N | | |

| Beispiel | A | B | D | X | Schmp/IR[$cm^{-1}$] | Isomer |
|---|---|---|---|---|---|---|
| 176 | 4-F-$C_6H_4$ | $OCH_3$ | Cl | N | | |
| 177 | 4-F-$C_6H_4$ | $SCH_3$ | Cl | N | | |
| 178 | 4-F-$C_6H_4$ | $SC_6H_5$ | Cl | N | | |
| 179 | 2-F-$C_6H_4$ | $OC_6H_5$ | Cl | N | | |
| 180 | 2-F-$C_6H_4$ | $OCH_3$ | Cl | N | | |
| 181 | 2-F-$C_6H_4$ | $SC_6H_5$ | Cl | N | | |
| 182 | 4-Cl-$C_6H_4$ | $OC_6H_5$ | Cl | N | | |
| 183 | 4-Cl-$C_6H_4$ | 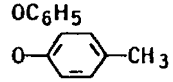 | Cl | N | | |
| 184 | 4-Cl-$C_6H_4$ | 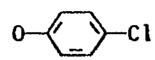 | Cl | N | | |
| 185 | 4-Cl-$C_6H_4$ | 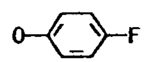 | Cl | N | | |
| 186 | 4-Cl-$C_6H_4$ | 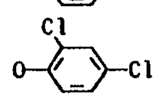 | Cl | N | | |
| 187 | 4-Cl-$C_6H_4$ | $OCH_3$ | Cl | N | | |
| 188 | 4-Cl-$C_6H_4$ | $SCH_3$ | Cl | N | | |
| 189 | 4-Cl-$C_6H_4$ | $SC_6H_5$ | Cl | N | | |
| 190 | 2-Cl-$C_6H_4$ | $OC_6H_5$ | Cl | N | | |
| 191 | 2-Cl-$C_6H_4$ | $OCH_3$ | Cl | N | | |
| 192 | 2-Cl-$C_6H_4$ | $SC_6H_5$ | Cl | N | | |
| 193 | 2,4-$Cl_2$-$C_6H_3$ | $OC_6H_5$ | Cl | N | | |
| 194 | 2,4-$Cl_2$-$C_6H_3$ | $OCH_3$ | Cl | N | | |
| 195 | 2,4-$Cl_2$-$C_6H_3$ | $SC_6H_5$ | Cl | N | | |

| Beispiel | A | B | D | X | Schmp/IR[$cm^{-1}$] | Isomer |
|---|---|---|---|---|---|---|
| 196 | 4-Br-$C_6H_4$ | $OCH_3$ | Cl | N | | |
| 197 | 4-$CH_3$-$C_6H_4$ | $OC_6H_5$ | Cl | N | | |
| 198 | 4-$CH_3$-$C_6H_4$ | $OCH_3$ | Cl | N | | |
| 199 | 4-$CH_3$-$C_6H_4$ | $SC_6H_5$ | Cl | N | | |
| 200 | 4-$OCH_3$-$C_6H_4$ | $OC_6H_5$ | Cl | N | | |
| 201 | 4-$OCH_3$-$C_6H_4$ | $OCH_3$ | Cl | N | | |
| 202 | 4-$OCH_3$-$C_6H_4$ | $SC_6H_5$ | Cl | N | | |
| 203 | 4-$CF_3$-$C_6H_4$ | $OC_6H_5$ | Cl | N | | |
| 204 | 4-$CF_3$-$C_6H_4$ | $OCH_3$ | Cl | N | | |
| 205 | 4-$CF_3$-$C_6H_4$ | $SC_6H_5$ | Cl | N | | |
| 206 | 4-Biphenyl | $OC_6H_5$ | Cl | N | | |
| 207 | 4-Biphenyl | $OCH_3$ | Cl | N | | |
| 208 | 4-Biphenyl | $SC_6H_5$ | Cl | N | | |
| 209 | 1-Naphthyl | $OCH_3$ | Cl | N | | |
| 210 | 2-Naphthyl | $OC_6H_5$ | Cl | N | | |
| 211 | 2-Naphthyl | $OCH_3$ | Cl | N | | |
| 212 | 2-Naphthyl | $SC_6H_5$ | Cl | N | | |
| 213 | Cyclopentyl | $OC_6H_5$ | Cl | N | | |
| 214 | Cyclopentyl | 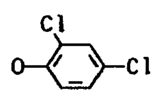 | Cl | N | | |
| 215 | Cyclopentyl | $OCH_3$ | Cl | N | | |
| 216 | Cyclopentyl | $SC_6H_5$ | Cl | N | | |

| Beispiel | A | B | D | X | Schmp/IR[$cm^{-1}$] | Isomer |
|---|---|---|---|---|---|---|
| 217 | Cyclohexyl | $OC_6H_5$ | Cl | N | | |
| 218 | Cyclohexyl | 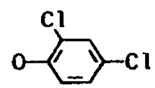 | Cl | N | | |
| 219 | Cyclohexyl | $OCH_3$ | Cl | N | | |
| 220 | Cyclohexyl | $SC_6H_5$ | Cl | N | | |
| 221 | 2-Thienyl | $CH_3$ | Cl | N | | |
| 222 | 2-Thienyl | $CH_3$ | 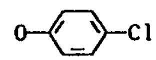 | N | | |
| 223 | 2-Thienyl | $C_2H_5$ | Cl | N | | |
| 224 | 2-Thienyl | $C_2H_5$ | 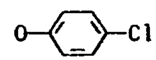 | N | | |
| 225 | 2-Thienyl | $C_3H_7$ | Cl | N | 2960,1506,1275,1135,701 | $D_1:D_2=3:2$ |
| 226 | 2-Thienyl | $C_3H_7$ | Cl | CH | 2959,1489.1226,1078,701 | $D_1:D_2=2:1$ |
| 227 | 2-Thienyl | $C_3H_7$ | $OCH_3$ | N | | |
| 228 | 2-Thienyl | $C_3H_7$ | 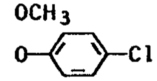 | N | | |
| 229 | 2-Thienyl | $C_3H_7$ | $SC_6H_5$ | N | | |
| 230 | 2-Thienyl | $C_3H_7$ | 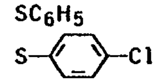 | N | | |
| 231 | 2-Thienyl | $C_6H_5$ | Cl | N | | |
| 232 | 2-Thienyl | $4-Cl-C_6H_4$ | Cl | N | | |
| 233 | 2-Thienyl | $2-Cl-C_6H_4$ | Cl | N | | |
| 234 | 2-Thienyl | $2,4-Cl_2-C_6H_3$ | Cl | N | | |
| 235 | 2-Thienyl | $4-F-C_6H_4$ | Cl | N | | |
| 236 | 2-Thienyl | $4-CH_3-C_6H_4$ | Cl | N | | |

| Beispiel | A | B | D | X | Schmp/IR[$cm^{-1}$] | Isomer |
|---|---|---|---|---|---|---|
| 237 | 2-Thienyl | 4-$OCH_3$-$C_6H_4$ | Cl | N | | |
| 238 | 2-Thienyl | 4-Biphenyl | Cl | N | | |
| 239 | 2-Thienyl | 1-Naphthyl | Cl | N | | |
| 240 | 2-Thienyl | 2-Naphthyl | Cl | N | | |
| 241 | 2-Thienyl | $CH_2$-$C_6H_5$ | Cl | N | | |
| 242 | 2-Thienyl | Cyclopentyl | Cl | N | | |
| 243 | 2-Thienyl | Cyclohexyl | Cl | N | | |
| 244 | 3-Thienyl | $CH_3$ | Cl | N | | |
| 245 | 3-Thienyl | $CH_3$ | 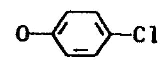 | N | | |
| 246 | 3-Thienyl | $C_2H_5$ | Cl | N | 2970, 1506, 1275, 1135, 778, 702 | $D_1$:$D_2$=2:1 |
| 247 | 3-Thienyl | $C_2H_5$ | Cl | CH | 86-88°C | $D_1$:$D_2$=2:1 |
| 248 | 3-Thienyl | $C_2H_5$ | $OCH_3$ | N | | |
| 249 | 3-Thienyl | $C_2H_5$ | 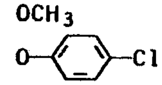 | N | | |
| 250 | 3-Thienyl | $C_2H_5$ | 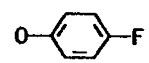 | N | | |
| 251 | 3-Thienyl | $C_2H_5$ | $SC_6H_5$ | N | 1502, 1439, 1274, 1137, 1011, 702 | $D_1$:$D_2$=2:1 |
| 252 | 3-Thienyl | $C_2H_5$ | $SC_6H_5$ | CH | 1501, 1476, 1274, 1136, 1095, 1013 | $D_1$:$D_2$=2:1 |
| 253 | 3-Thienyl | $C_2H_5$ | 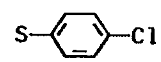 | N | | |
| 254 | 3-Thienyl | $C_3H_7$ | Cl | N | | |
| 255 | 3-Thienyl | $C_3H_7$ | $OCH_3$ | N | | |
| 256 | 3-Thienyl | $C_3H_7$ | 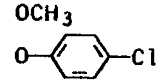 | N | | |

| Beispiel | A | B | D | X | Schmp/IR[$cm^{-1}$] | Isomer |
|---|---|---|---|---|---|---|
| 257 | 3-Thienyl | $C_3H_7$ | $SC_6H_5$ | N | | |
| 258 | 3-Thienyl | $C_3H_7$ | 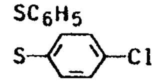 | N | | |
| 259 | 3-Thienyl | $C_6H_5$ | Cl | N | | |
| 260 | 3-Thienyl | $4-Cl-C_6H_4$ | Cl | N | | |
| 261 | 3-Thienyl | $2-Cl-C_6H_4$ | Cl | N | | |
| 262 | 3-Thienyl | $2,4-Cl_2-C_6H_3$ | Cl | N | | |
| 263 | 3-Thienyl | $4-F-C_6H_4$ | Cl | N | | |
| 264 | 3-Thienyl | $4-CH_3-C_6H_4$ | Cl | N | | |
| 265 | 3-Thienyl | $4-OCH_3-C_6H_4$ | Cl | N | | |
| 266 | 2-Furyl | $CH_3$ | Cl | N | | |
| 267 | 2-Furyl | $C_2H_5$ | Cl | N | | |
| 268 | 2-Furyl | $C_2H_5$ | Cl | CH | | |
| 269 | 2-Furyl | $C_2H_5$ | $OCH_3$ | N | | |
| 270 | 2-Furyl | $C_3H_7$ | Cl | N | | |
| 271 | 2-Furyl | $C_3H_7$ | $OCH_3$ | N | | |
| 272 | 2-Furyl | $C_3H_7$ | 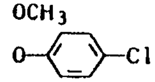 | N | | |
| 273 | 2-Furyl | $C_3H_7$ | $SC_6H_5$ | N | | |
| 274 | 2-Furyl | $C_6H_{13}$ | Cl | N | 2927, 1489, 1227, 1150, 1076, 733 | $D_1:D_2=1:1$ |
| 275 | 2-Furyl | $C_6H_{13}$ | Cl | CH | 2928, 1505, 1275, 1135, 1011, 734 | $D_1:D_2=2:1$ |
| 276 | 2-Furyl | $C_6H_{13}$ | $OCH_3$ | N | | |
| 277 | 2-Furyl | $C_6H_{13}$ | 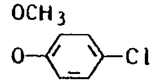 | N | | |

| Beispiel | A | B | D | X | Schmp/IR[$cm^{-1}$] | Isomer |
|---|---|---|---|---|---|---|
| 278 | 2-Furyl | $C_6H_13$ | $SC_6H_5$ | N | | |
| 279 | 2-Furyl | $C_6H_5$ | Cl | N | | |
| 280 | 2-Furyl | $4-Cl-C_6H_4$ | Cl | N | | |
| 281 | 2-Furyl | $2-Cl-C_6H_4$ | Cl | N | | |
| 282 | 2-Furyl | $4-F-C_6H_4$ | Cl | N | | |
| 283 | 2-Furyl | $2-F-C_6H_4$ | Cl | N | | |
| 284 | 2-Furyl | $4-Br-C_6H_4$ | Cl | N | | |
| 285 | 2-Furyl | $4-CH_3-C_6H_4$ | Cl | N | | |
| 286 | 2-Furyl | $4-OCH_3-C_6H_4$ | Cl | N | | |
| 287 | 2-Pyridyl | $CH_3$ | Cl | N | | |
| 288 | 2-Pyridyl | $C_2H_5$ | Cl | N | | |
| 289 | 2-Pyridyl | $C_2H_5$ | Cl | CH | | |
| 290 | 2-Pyridyl | $C_2H_5$ | $OCH_3$ | N | | |
| 291 | 2-Pyridyl | $C_2H_5$ | 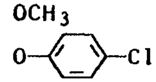 | N | | |
| 292 | 2-Pyridyl | $C_3H_7$ | Cl | N | | |
| 293 | 2-Pyridyl | $C_3H_7$ | Cl | CH | | |
| 294 | 2-Pyridyl | $C_3H_7$ | $OCH_3$ | N | | |
| 295 | 2-Pyridyl | $C_3H_7$ | 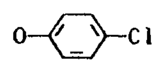 | N | | |
| 296 | 2-Pyridyl | $C_6H_5$ | Cl | N | | |
| 297 | 2-Pyridyl | $4-Cl-C_6H_4$ | Cl | N | | |
| 298 | 2-Pyridyl | $2-Cl-C_6H_4$ | Cl | N | | |
| 299 | 2-Pyridyl | $2-F-C_6H_4$ | Cl | N | | |

| Beispiel | A | B | D | X | Schmp/IR[$cm^{-1}$] | Isomer |
|---|---|---|---|---|---|---|
| 300 | 2-Pyridyl | $4-F-C_6H_4$ | Cl | N | | |
| 301 | 2-Pyridyl | $4-Br-C_6H_4$ | Cl | N | | |
| 302 | 2-Pyridyl | $4-CH_3-C_6H_4$ | Cl | N | | |
| 303 | 2-Pyridyl | $4-OCH_3-C_6H_4$ | Cl | N | | |
| 304 | 3-Pyridyl | $CH_3$ | Cl | N | | |
| 305 | 3-Pyridyl | $CH_3$ | Cl | CH | | |
| 306 | 3-Pyridyl | $C_2H_5$ | Cl | N | | |
| 307 | 3-Pyridyl | $C_2H_5$ | Cl | CH | | |
| 308 | 3-Pyridyl | $C_2H_5$ | $OCH_3$ | N | | |
| 309 | 3-Pyridyl | $C_2H_5$ | 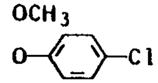 | N | | |
| 310 | 3-Pyridyl | $C_2H_5$ | $SC_6H_5$ | N | | |
| 311 | 3-Pyridyl | $C_3H_7$ | Cl | N | | |
| 312 | 3-Pyridyl | $C_3H_7$ | Cl | CH | | |
| 313 | 3-Pyridyl | $C_3H_7$ | $OCH_3$ | N | | |
| 314 | 3-Pyridyl | $C_3H_7$ | 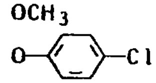 | N | | |
| 315 | 3-Pyridyl | $C_3H_7$ | 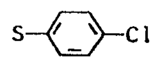 | N | | |
| 316 | 3-Pyridyl | $C_6H_5$ | Cl | N | | |
| 317 | 3-Pyridyl | $4-Cl-C_6H_4$ | Cl | N | | |
| 318 | 3-Pyridyl | $2-Cl-C_6H_4$ | Cl | N | | |
| 319 | 3-Pyridyl | $4-F-C_6H_4$ | Cl | N | | |

| Beispiel | A | B | D | X | Schmp/IR[$cm^{-1}$] | Isomer |
|---|---|---|---|---|---|---|
| 320 | 3-Pyridyl | 2-F-$C_6H_4$ | Cl | N | | |
| 321 | 3-Pyridyl | 4-Br-$C_6H_4$ | Cl | N | | |
| 322 | 3-Pyridyl | 4-$CH_3$-$C_6H_4$ | Cl | N | | |
| 323 | 3-Pyridyl | 4-$OCH_3$-$C_6H_4$ | Cl | N | | |
| 324 | 4-Pyridyl | $CH_3$ | Cl | N | | |
| 325 | 4-Pyridyl | $CH_3$ | Cl | CH | | |
| 326 | 4-Pyridyl | $C_2H_5$ | Cl | N | | |
| 327 | 4-Pyridyl | $C_2H_5$ | Cl | CH | | |
| 328 | 4-Pyridyl | $C_2H_5$ | $OCH_3$ | N | | |
| 329 | 4-Pyridyl | $C_2H_5$ | 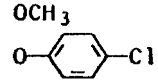 | N | | |
| 330 | 4-Pyridyl | $C_2H_5$ | $SC_6H_5$ | N | | |
| 331 | 4-Pyridyl | $C_3H_7$ | Cl | N | | |
| 332 | 4-Pyridyl | $C_3H_7$ | Cl | CH | | |
| 333 | 4-Pyridyl | $C_3H_7$ | $OCH_3$ | N | | |
| 334 | 4-Pyridyl | $C_3H_7$ | 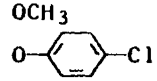 | N | | |
| 335 | 4-Pyridyl | $C_3H_7$ | $SC_6H_5$ | N | | |
| 336 | 4-Pyridyl | $C_6H_5$ | Cl | N | | |
| 337 | 4-Pyridyl | 4-Cl-$C_6H_4$ | Cl | N | | |
| 338 | 4-Pyridyl | 2-Cl-$C_6H_4$ | Cl | N | | |
| 339 | 4-Pyridyl | 4-F-$C_6H_4$ | Cl | N | | |
| 340 | 4-Pyridyl | 2-F-$C_6H_4$ | Cl | N | | |
| 341 | 4-Pyridyl | 4-Br-$C_6H_4$ | Cl | N | | |

| Bei-spiel | A | B | D | X | Schmp/IR[$cm^{-1}$] | Isomer |
|---|---|---|---|---|---|---|
| 342 | 4-Pyridyl | 4-$CH_3$-$C_6H_4$ | Cl | N | | |
| 343 | 4-Pyridyl | 4-$OCH_3$-$C_6H_4$ | Cl | N | | |
| 344 | 4-Oxazolyl | $CH_3$ | Cl | N | | |
| 345 | 4-Oxazolyl | $C_2H_5$ | Cl | N | | |
| 346 | 5-Oxazolyl | $CH_3$ | Cl | N | | |
| 347 | 5-Oxazolyl | $C_2H_5$ | Cl | N | | |
| 348 | 5-Oxazolyl | $C_6H_5$ | Cl | N | | |
| 349 | 3-Isoxazolyl | $CH_3$ | Cl | N | | |
| 350 | 3-Isoxazolyl | $C_2H_5$ | Cl | N | | |
| 351 | 3-Isoxazolyl | $C_6H_5$ | Cl | N | | |
| 352 | 4-Isoxazolyl | $CH_3$ | Cl | N | | |
| 353 | 4-Isoxazolyl | $C_2H_5$ | Cl | N | | |
| 354 | 4-Isoxazolyl | $C_6H_5$ | Cl | N | | |
| 355 | 5-Isoxazolyl | $CH_3$ | Cl | N | | |
| 356 | 5-Isoxazolyl | $C_2H_5$ | Cl | N | | |
| 357 | 5-Isoxazolyl | $C_6H_5$ | Cl | N | | |
| 358 | 4-Thiazolyl | $CH_3$ | Cl | N | | |
| 359 | 4-Thiazolyl | $C_2H_5$ | Cl | N | | |
| 360 | 4-Thiazolyl | $C_6H_5$ | Cl | N | | |
| 361 | 5-Thiazolyl | $CH_3$ | Cl | N | | |
| 362 | 5-Thiazolyl | $C_2H_5$ | Cl | N | | |
| 363 | 5-Thiazolyl | $C_6H_5$ | Cl | N | | |

| Beispiel | A | B | D | X | Schmp/IR[cm$^{-1}$] | Isomer |
|---|---|---|---|---|---|---|
| 364 | 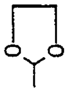 | $CH_3$ | Cl | N | | |
| 365 | 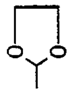 | $C_6H_5$ | Cl | N | | |
| 366 | $CH_2CH_2CO_2CH_3$ | $C_6H_5$ | Cl | N | 1729, 1506, 1276, 1202, 1135, 702 cm-1 | $D_1:D_2=3:1$ |
| 367 | $CH_2CH_2CO_2CH_3$ | $C_6H_5$ | Cl | CH | | |
| 368 | $CH_2CH_2CO_2CH_3$ | $C_6H_5$ | $OCH_3$ | N | | |
| 369 | $CH_2CH_2CO_2CH_3$ | $C_6H_5$ | $SC_6H_5$ | N | | |
| 370 | $CH_3$ | 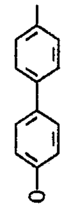 | Cl | N | Harz | $D_1:D_2=3:1$ |

$D_1:D_2$ = Verhältnis der gebildeten Diastereomeren

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis,

Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzbefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 10 g, je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 9 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 16 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 80 werden in einer Mischung gelöst, die aus 40 Gew.-Teilen Cyclohexanon, 30 Gew.-Teilen Isobutanol, 20 Gew.-Teilen des Anlagerungsproduktes von 40 Mol

Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 225 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 226 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 246 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 247 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 251 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 252 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen
eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoffe wurden 1-(1,2,4-Triazol-1-yl)-1-phenylthio-2-methyl-2-phenylpropan (A) - bekannt aus EP 91 219 -, 1-(1,2,4-Triazol-1-yl)1-(4-chlorphenylthio)-2-methyl-2-phenylpropan (B) - bekannt aus EP 91 219 und 1-Methoxy-1-(1,2,4-triazol-1-yl)-2-phenylethan (C) - bekannt aus DE 2 640 823 - benutzt.

Anwendungsbeispiel 1

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in einer Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß die Wirkstoffe 2, 80 und 247 bei der Anwendung als 0,025 %ige (Gew.%) Spritzbrühe eine bessere fungizide Wirkung zeigen (90 %) als die bekannten Vergleichswirkstoffe A, B und C (40 %).

Anwendungsbeispiel 2

Wirksamkeit gegen Botrytis cinerea

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer

Konidienaufschwemmung des Pilzes Botrytis cinerea besprüht und bei 22 bis 24° C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedecken. Es wurde der Befall der Blätter geprüft.

Das Ergebnis zeigt, daß die Wirkstoffe 225 und 247 bei der Anwendung als 0,05 %ige (Gew.-%) Spritzbrühe eine bessere fungizide Wirkung zeigen (85 %) als die bekannten Vergleichswirkstoffe A, B und C (10 %).

Anwendungsbeispiel 3

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20 bis 22°C und 70 % relativer Luftfeuchtigkeit für weitere 5 Tage kultiviert. Dann wurde das Ausmaß des Blattbefalls ermittelt.

Das Ergebnis zeigt, daß die Wirkstoffe 9, 16, 80, 225, 226, 246, 247, 251 und 252 bei der Anwendung als 0,05 %ige (Gew.-%) Spritzbrühe eine bessere fungizide Wirkung zeigen (95 %) als die bekannten Vergleichswirkstoffe A, B und C (30 %).

**Patentansprüche**

1. Azolylethanderivate der allgemeinen Formel I

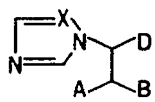

I

in welcher
A und B gleich oder verschieden sind und $C_1$-$C_8$-Alkyl, Phenyl, Biphenyl, Naphthyl, Benzyl, $C_3$-$C_6$-Cycloalkyl, $C_1$-$C_8$-Alkoxy, $C_1$-$C_8$-Alkylthio, Phenoxy, Benzyloxy, Biphenyloxy, Phenylthio, Methoxycarbonylethyl oder 5- oder 6-gliedriges Heteroaryl bedeuten, wobei diese Reste ein- bis dreifach durch Halogen, Nitro, Phenoxy, $C_1$-$C_4$-Alkyl, $C_1$-$C_4$-Alkoxy oder $C_1$-$C_4$-Halogenalkyl substituiert sein können, mit der Maßgabe, daß mindestens einer der Substituenten A und B $C_1$-$C_8$-Alkoxy, $C_1$-$C_8$-Alkylthio, Phenoxy, Benzyloxy, Phenylthio oder 5- oder 6-gliedriges Heteroaryl bedeutet,
D Halogen, $C_1$-$C_4$-Alkoxy, $C_1$-$C_4$-Alkylthio, Phenylthio oder Phenoxy bedeutet, wobei diese Reste einfach bis dreifach durch Halogen, Nitro, Phenoxy, $C_1$-$C_4$-Alkyl oder $C_1$-$C_4$-Halogenalkyl substituiert sein können,
X den Rest CH oder N bedeutet,
sowie ihre pflanzenverträglichen Säureadditionssalze und Metallkomplexverbindungen.

2. Verfahren zur Herstellung der Azolylethanderivate der Formel I,

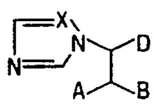

I

in welcher
A und B gleich oder verschieden sind und $C_1$-$C_8$-Alkyl, Phenyl, Biphenyl, Naphthyl, Benzyl, $C_3$-$C_6$-Cycloalkyl, $C_1$-$C_8$-Alkoxy, $C_1$-$C_8$-Alkylthio, Phenoxy, Benzyloxy, Biphenyloxy, Phenylthio, Methoxycarbonylethyl oder 5- oder 6-gliedriges Heteroaryl bedeuten, wobei diese Reste ein- bis dreifach durch Halogen, Nitro, Phenoxy, $C_1$-$C_4$-Alkyl, $C_1$-$C_5$-Alkoxy oder $C_1$-$C_4$-Halogenalkyl substituiert sein können, mit der Maßgabe, daß mindestens einer der Substituenten A und B $C_1$-$C_8$-Alkoxy, $C_1$-$C_8$-Alkylthio, Phenoxy, Benzyloxy, Phenylthio oder 5- oder 6-gliedriges Heteroaryl bedeutet,

D Chlor oder Brom bedeutet,
X den Rest CH oder N bedeutet,
sowie ihre pflanzenverträglichen Säureadditionssalze und Metallkomplexverbindungen,dadurch gekennzeichnet, einen Aldehyd der Formel II

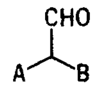

II

in welcher A und B die in Anspruch 1 angegebenen Bedeutungen haben, mit einem Azol der Formel III

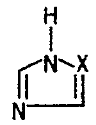

III

in Gegenwart von anorganischen Säurechloriden oder -bromiden umsetzt und die so erhaltenen Verbindungen gewünschtenfalls in ihre Salze oder Metallkomplexe überführt.

3. Verfahren zur Herstellung der Azolylethanderivate der Formel I gemäß Anspruch 1, in der D $C_1$-$C_4$-Alkoxy bedeutet, dadurch gekennzeichnet, daß man ein Acetal der Formel IV

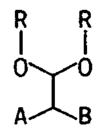

IV

in welcher A und B die in Anspruch 1 angegebene Bedeutung haben und R den Rest $C_1$-$C_4$-Alkyl bedeutet, mit anorganischen oder organischen Säurechloriden und anschließend mit einem Azol der Formel III umsetzt und die erhaltenen Verbindungen gewünschtenfalls in ihre Salze oder Metallkomplexe überführt.

4. Verfahren zur Herstellung der Azolylethanderivate der Formel I gemäß Anspruch 1, in der D für einen anderen Substituenten als Chlor, Brom oder $C_1$-$C_4$-Alkoxy steht, dadurch gekennzeichnet, daß man ein Azolylethanderivat der Formel Ia

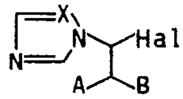

Ia

in der Hal Chlor oder Brom bedeutet, mit einer Verbindung der Formel D-H und einer Base umsetzt und die so erhaltenen Verbindungen gewünschtenfalls in ihre Salze oder Metallkomplexe überführt.

5. Fungizides Mittel, enthaltend einen inerten Trägerstoff und eine fungizid wirksame Menge eines Azolylethanderivats der Formel I,

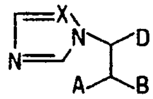

I

in welcher
A und B gleich oder verschieden sind und $C_1$-$C_8$-Alkyl, Phenyl, Biphenyl, Naphthyl, Benzyl, $C_3$-$C_6$-Cycloalkyl, $C_1$-$C_8$-Alkoxy, $C_1$-$C_8$-Alkylthio, Phenoxy, Benzyloxy, Biphenyloxy, Phenylthio, Methoxycarbonylethyl oder 5- oder 6-gliedriges Heteroaryl bedeuten, wobei diese Reste ein- bis dreifach durch

Halogen, Nitro, Phenoxy, $C_1$-$C_4$-Alkyl, $C_1$-$C_4$-Alkoxy oder $C_1$-$C_4$-Halogenalkyl substituiert sein können, mit der Maßgabe, daß mindestens einer der Substituenten A und B $C_1$-$C_8$-Alkoxy, $C_1$-$C_8$-Alkylthio, Phenoxy, Benzyloxy, Phenylthio oder 5- oder 6-gliedriges Heteroaryl bedeutet,
D Halogen, $C_1$-$C_4$-Alkoxy, $C_1$-$C_4$-Alkylthio, Phenylthio oder Phenoxy bedeutet, wobei diese Reste einfach bis dreifach durch Halogen, Nitro, Phenoxy, $C_1$-$C_4$-Alkyl oder $C_1$-$C_4$-Halogenalkyl substituiert sein können,
X den Rest CH oder N bedeutet,
oder dessen pflanzenverträglichen Salzes oder Metallkomplexes.

**6.** Verfahren zur Bekämpfung von Pilzen, dadurch gekennzeichnet, daß man eine fungizid wirksame Menge eines Azolylethanderivates der Formel I,

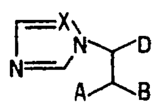

I

in welcher
A und B gleich oder verschieden sind und $C_1$-$C_8$-Alkyl, Phenyl, Biphenyl, Naphthyl, Benzyl, $C_3$-$C_6$-Cycloalkyl, $C_1$-$C_8$-Alkoxy, $C_1$-$C_8$-Alkylthio, Phenoxy, Benzyloxy, Biphenyloxy, Phenylthio, Methoxycarbonylethyl oder 5- oder 6-gliedriges Heteroaryl bedeuten, wobei diese Reste ein- bis dreifach durch Halogen, Nitro, Phenoxy, $C_1$-$C_4$-Alkyl, $C_1$-$C_4$-Alkoxy oder $C_1$-$C_4$-Halogenalkyl substituiert sein können, mit der Maßgabe, daß mindestens einer der Substituenten A und B $C_1$-$C_8$-Alkoxy, $C_1$-$C_8$-Alkylthio, Phenoxy, Benzyloxy, Phenylthio oder 5- oder 6-gliedriges Heteroaryl bedeutet,
D Halogen, $C_1$-$C_4$-Alkoxy, $C_1$-$C_4$-Alkylthio, Phenylthio oder Phenoxy bedeutet, wobei diese Reste einfach bis dreifach durch Halogen, Nitro, Phenoxy, $C_1$-$C_4$-Alkyl oder $C_1$-$C_4$-Halogenalkyl substituiert sein können,
X den Rest CH oder N bedeutet,
dessen pflanzenverträglichen Salzes oder Metallkomplexes auf Pilze oder vom Pilzbefall bedrohte Pflanzen, deren Lebensraum, Materialien, Holz oder Saatgut einwirken läßt.

**7.** Verbindung der Formel I gemäß Anspruch 1, dadurch gekennzeichnet, daß A Methyl, B 4-Methylphenoxy, D Cl und X N bedeuten.

**8.** Verbindung der Formel I gemäß Anspruch 1, dadurch gekennzeichnet, daß A Methyl, B 2,4-Dichlorphenoxy, D C1 und X N bedeuten.